# EUROPEAN PATENT APPLICATION

(11) **EP 1 985 364 A1**
(43) Date of publication of application: **29.10.2008**
(21) Application number: 07714397.2
(22) Date of filing: 16.02.2007
(51) Int. Cl.: B01J 31/28, B01J 37/03, C07C 29/149, C07C 31/04, C07B 61/00, C07C 27/22, C07C 67/36, C07C 69/06

(54) **METHANOL SYNTHESIS CATALYST, METHOD FOR PRODUCING SUCH CATALYST AND METHOD FOR PRODUCING METHANOL**

(30) Priority: 17.02.2006 JP 2006041618; 31.01.2007 JP 2007022125
(71) Applicant: Nippon Steel Engineering Co., Ltd, Tokyo 100-8071 (JP)
(72) Inventor: FUJIMOTO, Kaoru, Kitakyushu-shi, Fukuoka 808-0135 (JP); FUJIMOTO, Kenichiro, Futtsu-shi, Chiba 293-8511 (JP); YAMANE, Noriyuki, Futtsu-shi, Chiba 293-8511 (JP)
(74) Representative: Vossius & Partner
(86) International application number: PCT/JP2007/052867
(87) International publication number: WO 2007/094461

(57) **Abstract**

This catalyst for methanol synthesis, where methanol is synthesized via a formic ester, is a catalyst for methanol synthesis which carries out a reaction under the presence of a starting material gas containing hydrogen and at least either one of carbon monoxide and carbon dioxide, and an alcohol as a solvent, and includes a catalyst containing Cu, Mg, Na, Pd, and an alkali metal formate salt.

## Description

### TECHNICAL FIELD

The present invention relates to a catalyst for methanol synthesis, a method for producing the catalyst, and a method for producing methanol. More specifically, the present invention relates to a catalyst which is highly active when producing methanol from hydrogen and a carbon source, i.e., either carbon monoxide or carbon dioxide, and a method for obtaining a product with high efficiency using this catalyst
Priority is claimed on Japanese Patent Application No. 2006-41618, filed February 17, 2006, and Japanese Patent Application No. 2007-22125, filed January 31, 2007, the contents of which are incorporated herein by reference.

### BACKGROUND ART

Generally, in the industrial synthesis of methanol, carbon monoxide and hydrogen (synthesis gas) obtained by steam reforming of a natural gas mainly composed of methane are used as the starting materials and the synthesis is carried out by a fixed-bed gas phase process using a copper/zinc-based catalyst or the like under severe conditions of a temperature of 200 to 300°C and a pressure of 5 to 25 MPa (Non-patent Document 1). A generally accepted explanation for the reaction mechanism is that it is a successive reaction where methanol and water are first produced due to the hydrogenation of carbon dioxide and then the produced water reacts with carbon monoxide to produce carbon dioxide and hydrogen (water-gas shift reaction).

CO₂ + 3H₂ → CH₃OH + H₂O (1)

H₂O + CO → CO₂ + H₂ (2)

CO + 2H₂ → CH₃OH (3)

Although this reaction is an exothermic reaction, efficient heat extraction is difficult to achieve because of poor thermal conductivity in the gas phase method, and a process of lowering the one-pass conversion and recycling unreacted high-pressure starting material gas is employed, which has, however, a severe problem in the efficiency. Despite the above problem, the fixed-bed gas phase method is not easily prone to the reaction inhibition by water or carbon dioxide contained in the synthesis gas and various plants are now operated by making use of this advantageous property.

On the other hand, various methods of synthesizing methanol in a liquid phase and thereby increasing the heat extraction rate have been studied. Among them, a method of using a catalyst having high activity at low temperatures (approximately from 100 to 180°C) is also thermodynamically advantageous for the production system, and thus drawing attention (Non-patent Document 2 and the like). However, it has been reported that water and carbon dioxide, which are always contained in the synthesis gas, decrease the catalytic activity in these methods, and thus none of them has been put into practice, although they use an alkali metal alkoxide as a catalyst (Non-patent Document 3). This is because the highly active alkali metal alkoxide changes into a low active, stable formate salt or the like during the reaction. In order to prevent the decrease of the catalytic activity, water and carbon dioxide in the starting material gas need to be removed down to the order of ppb. However, this is not realistic, since the production cost increases if such a pretreatment is carried out.

To date, the present inventors have discovered the following system (Patent Document 1), in which, as the catalyst whose catalytic activity is little decreased due to water and carbon dioxide, one or both of a catalyst based on an alkali metal except alkali metal alkoxide and an alkaline earth metal-based catalyst are used under the presence of a hydrogenolysis catalyst. The above Patent Document 1 describes Cu/Mn, Cu/Re, Cu/MgO, and the like as effective hydrogenolysis catalysts. However, the present inventors discovered a Cu-based catalyst having much higher activity than those of the above hydrogenolysis catalysts in their later studies.
[Patent Document 1] Japanese Unexamined Patent Application, First Publication No. 2001-862701
[Non-patent Document 1] J. C. J. Bart et al., Catal. Today, 2, 1 (1987)
[Non-patent Document 2] Seiichi Ohyama, Petrotech, 18(1), 27 (1995)
[Non-patent Document 3] S. Ohyama, Applied Catalysis A: General, 180, 217 (1999)

### DISCLOSURE OF INVENTION

### PROBLEMS TO BE SOLVED BY THE INVENTION

An object of the present invention is to solve the above problems and to provide a catalyst whose catalytic activity decreases only slightly even when a small amount of carbon dioxide, water, and the like are present in the starting material gas for methanol synthesis and which is also capable of synthesizing a formic ester and methanol under low temperature and pressure conditions, a method for producing the catalyst, and a method for synthesizing methanol in the liquid phase using the catalyst. MEANS FOR SOLVING THE PROBLEMS

The present invention is characterized by the following aspects.
Aspect (1) is a catalyst for methanol synthesis, where methanol is synthesized via a formic ester, which carries out a reaction under the presence of a starting material gas containing hydrogen and at least either one of carbon monoxide and carbon dioxide, and an alcohol as a solvent, the catalyst for methanol synthesis including, in addition to an alkali metal formate salt, a catalyst containing Cu, Mg, Na, and Pd.

Aspect (2) is the catalyst for methanol synthesis according to aspect (1), wherein the alkali metal formate salt is potassium formate.

Aspect (3) is a catalyst for methanol synthesis, where methanol is synthesized via a formic ester, which carries out a reaction under the presence of a starting material gas containing hydrogen and at least either one of carbon monoxide and carbon dioxide, and an alcohol as a solvent, the catalyst for methanol synthesis containing Cu, Mg, Na, and Pd in addition to an alkali metal-based catalyst which can change into an alkali metal formate salt.

Aspect (4) is the catalyst for methanol synthesis according to aspect (3), wherein the alkali metal-based catalyst which can change into an alkali metal formate salt is an alkali metal carbonate salt.

Aspect (5) is the catalyst for methanol synthesis according to any one of aspects (1) to (4), wherein said Na is loaded as a carbonate salt or a formate salt on a Cu/MgO solid catalyst.

Aspect (6) is the catalyst for methanol synthesis according to any one of aspects (1) to (5), wherein said Pd is loaded on a Cu/MgO solid catalyst.

Aspect (7) is the catalyst for methanol synthesis according to any one of aspects (1) to (6), wherein a loading amount of Pd is 0.001 to 1 mass% based on the catalyst containing Cu, Mg, Na, and Pd.

Aspect (8) is a method for producing the catalyst for methanol synthesis according to any one of aspects (5) to (7), the method including the steps of preparing a Cu/MgO solid catalyst, and loading Na and Pd on the solid catalyst.

Aspect (9) is the method for producing the catalyst for methanol synthesis according to any one of aspects (5) to (7), including the steps of preparing said Cu/MgO by coprecipitation method, and loading Na and Pd on said Cu/MgO by impregnation method.

Aspect (10) is a method for producing the catalyst for methanol synthesis according to any one of aspects (5) to (7), including the step of preparing wherein said Cu/MgO by coprecipitation method while maintaining a constant pH within a range of 8 to 11.

Aspect (11) is a method for producing methanol including the steps of reacting a starting material gas containing hydrogen and at least either one of carbon monoxide and carbon dioxide under the presence of the catalyst described in any one of aspects (1) to (7) and an alcohol, thereby producing a formic ester and methanol; and hydrogenating the formic ester thus obtained to produce methanol.

Aspect (12) is a method for producing methanol including the steps of reacting a starting material gas containing hydrogen and at least either one of carbon monoxide and carbon dioxide under the presence of the catalyst described in any one of aspects (1) to (7) and an alcohol; separating products thus obtained from a reaction system; and hydrogenating the formic ester in the products by using a hydrogenolysis catalyst to produce methanol.

Aspect (13) is the method for producing methanol according to aspect (11) or (12), wherein an alkali metal-based catalyst which can change into an alkali metal formate salt during reaction is used instead of the alkali metal formate salt.

Aspect (14) is the method for producing methanol according to any one of aspects (11) to (13), wherein the alkali metal formate salt is potassium formate.

Aspect (15) is the method for producing methanol according to aspect (14), wherein the alkali metal-based catalyst which can change into an alkali metal formate salt during reaction is an alkali metal carbonate salt

Aspect (16) is the method for producing methanol according to any one of aspects (11) to (15), wherein the alcohol is a primary alcohol.

### EFFECTS OF THE INVENTION

When methanol is produced in a system of the present invention where a catalyst containing Cu, Mg, Na, and Pd coexists with an alkali metal formate salt under the presence of a synthesized starting material gas, which contains hydrogen and at least one of carbon monoxide and carbon dioxide, and a solvent alcohol, it is possible to synthesize methanol stably at high efficiency in a continuous reaction under low temperature and pressure conditions. Moreover, it is possible to produce methanol at low cost since the extent of decrease of the catalytic activity remains low even when a small amount of water, carbon dioxide, or the like is mixed in the synthesized starting material gas.

### BRIEF DESCRIPTION OF THE DRAWING

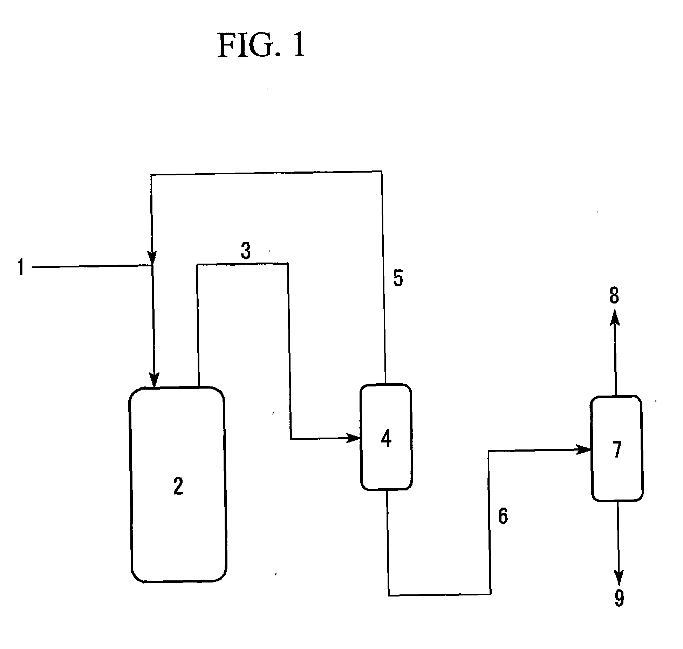

FIG. 1 shows a reactor for conducting liquid phase synthesis of methanol at low temperatures according to the present invention.

### DESCRIPTION OF THE REFERENCE SYMBOLS

- 1: Synthesis gas
- 2: Semi-batch reactor
- 3: Mixture of product and unreacted gas
- 4: Cooler
- 5: Unreacted gas
- 6: Liquid mixture of formic ester and methanol
- 7: Distillation column
- 8: Formic ester
- 9: Methanol

### BEST MODE FOR CARRYING OUT THE INVENTION

The present invention will be described in detail below.
As a result of intensive studies, the present inventors discovered the following to complete the present invention. That is, in the continuous reaction using a semi-batch system where a catalyst and a solvent are fed to a reactor and a starting material gas is also supplied thereto, it is possible to produce high yield of methanol from at least one of carbon monoxide and carbon dioxide, hydrogen, and an alcohol, when a catalyst containing Cu, Mg, Na, and Pd is used in addition to an alkali metal formate salt.

For example, methanol can be produced continuously by the reaction process shown in FIG. 1. In addition to an alkali metal formate salt, a solid catalyst containing Cu, Mg, Na, and Pd is fed with a solvent alcohol to a semi-batch reactor 2, and then synthesis gas 1 is supplied thereto. A mixture 3 of products (i.e., formic ester and methanol) and unreacted gas collected at the reactor outlet is cooled in a cooler 4 to be separated into unreacted gas 5 and a liquid mixture 6 of a formic ester and an alcohol. The latter mixture is further separated into a formic ester 8 and methanol 9 in a distillation column 7 provided in the next step. When the conversion is low, it is possible to supply the unreacted gas again to the semi-batch reactor 2. However, when the product is obtained at a high yield, the unreacted gas is used as a heat source (fuel) in the production of synthesis gas.

Examples of the alkali metal formate salt include potassium formate, sodium formate, cesium formate, and rubidium formate. Potassium formate is particularly preferable since its use enhances the catalytic activity.

In addition, it is also possible to use an alkali metal-based catalyst, which can adopt the form of a formate salt during the reaction, instead of the alkali metal formate salt, and the form thereof at the time of being fed to the reaction is not particularly limited.

Examples of such alkali metal-based catalysts include potassium carbonate and potassium methoxide. When potassium carbonate is used, it is postulated that potassium carbonate changes into potassium formate by the following reaction. Even when the alkali metal-based catalyst is fed in other forms, it is assumed that the catalyst changes into a formate salt, which is more stable.

K₂CO₃ + H₂O → 2KOH + CO₂ (4)

KOH + CO → HCOOK (5)

Specifically, the solid catalyst which coexists with the abovementioned alkali metal formate salt or the alkali metal-based catalyst capable of changing into an alkali metal formate salt is Cu/MgO_{X}/Na/Pd (wherein, X is a chemically allowable value) and examples thereof include Cu/MgO_{X}/HCOONa/Pd (wherein, X is a chemically allowable value). The method for preparing Cu/MgO_{X} is not particularly limited and an ordinary method such as impregnation method, precipitation method, sol-gel method, coprecipitation method, ion exchange method, kneading method and drying method may be used, although good results are readily obtained by the use of coprecipitation method. CO conversion varies considerably depending on the pH, which is maintained constant when preparing a catalyst with coprecipitation method. The pH when preparing the Cu/MgO_{X} catalyst is preferably 8 to 11, more preferably 8.5 to 10.5, and even more preferably 9 to 10. The pH range exceeding 11 is not economical since the amount of an alkaline compound used as a precipitant in order to maintain a highly alkaline atmosphere increases distinctively. The method for loading an Na salt on Cu/MgO_{X} is not particularly limited and the abovementioned ordinary methods may be used, although good results are readily obtained by the use of impregnation method or drying method. The loading amount of Na relative to Cu/MgO_{X} is not particularly limited as long as it is at least the minimum amount where its effects are exhibited. However, the amount is preferably within a range of 0.1 to 60 mass%, more preferably 1 to 40 mass%, and even more preferably 3 to 30 mass%. In addition, the loaded Na salt is preferably sodium formate, sodium carbonate, or the like. Catalytic activity increases when these Na salts are loaded. Moreover, Cu/MgO_{X}/Na can suppress the decrease of the catalytic activity over time which is observed slightly in the Cu/MgO_{X} catalysts. Accordingly, it can be said that the addition of the alkali metal carbonate salt is effective for improving catalytic activity and for suppressing the decrease of catalytic activity.

The method for loading Pd is not particularly limited and the ordinary methods may be used, although good results are readily obtained by the use of impregnation method or drying method as described earlier. The loading amount of Pd relative to Cu/MgO_{X}/Na is not particularly limited as long as it is at least the minimum amount where its effects are exhibited. However, the amount is preferably within a range of 0.001 to 1 mass%, more preferably 0.005 to 0.5 mass%, and even more preferably 0.01 to 0.1 mass%. Catalytic activity increases when Pd is loaded.

Although it is preferable to sequentially load Na and Pd on Cu/MgO_{X} as described above, it is also possible to simultaneously load Na and Pd in the case that the loaded Na salt and the precursor of Pd dissolve in the same liquid. Additionally, it is also possible to load Pd first on Cu/MgO_{X} thereby preparing Cu/MgO_{X}/Pd, and then to load the Na salt thereon.

The abovementioned solid catalyst containing Cu, Mg, Na, and Pd exhibits a catalytic action mainly in the hydrogenolysis of the produced formic esters, although a catalytic action thereof is also exhibited in the reaction for inserting CO in a solvent alcohol.

The alcohol used in the reaction may be an alcohol where a hydroxyl group is bonded with a chained or an alicyclic hydrocarbon, phenol or a substitution product thereof, or thiol or a substitution product thereof. Although these alcohols may be any of primary, secondary and tertiary alcohols, primary alcohols are preferable due to their reaction efficiency. Lower alcohols such as methyl alcohols and ethyl alcohols are most commonly used.

Although the reaction can be performed in either the liquid phase or the gas phase, a system where moderate conditions can be selected may be employed. Specifically, preferable conditions are the temperature of 70 to 250°C and the pressure of 3 to 100 atmospheres, more preferably the temperature of 120 to 200°C and the pressure of 15 to 80 atmospheres, although conditions are not limited to the above. Although the amount of alcohol used is not limited as long as it is sufficient enough for the reaction to proceed, even larger amount of alcohol may be used as a solvent. In addition, an organic solvent other than alcohols may be used appropriately in combination during the above reaction.

The obtained formic ester may be purified by an ordinary method such as distillation, but may also be used as it is in the production of methanol. In other words, methanol can be produced by hydrogenating the formic ester.

A hydrogenolysis catalyst is used in the hydrogenolysis process. For example, a common hydrogenolysis catalyst which is based on Cu, Pt, Ni, Co, Ru, and Pd may be employed, but Cu/MgO_{X}/Na/Pd of the present invention can also be used. By making these common hydrogenolysis catalysts coexist in the aforementioned reaction system where a formic ester and methanol are produced from the starting material gas and an alcohol, the selectivity for methanol increases, and thus methanol can be produced efficiently.

In addition, when it is difficult to produce methanol in the one-step process under a reaction condition where the selectivity for formic esters is high, it is also possible to obtain methanol by first separating the products obtained in the reaction from the reaction system using a distillation method or the like and then hydrogenating the formic ester in the products in the presence of a hydrogenolysis catalyst and hydrogen.

Although methanol can be obtained with the method using the catalyst of the present invention even if CO₂ is the only carbon source in the starting material gas, the catalytic activity is lower compared to that if CO is the sole carbon source in the starting material gas. In addition, the lower the concentrations of CO₂ and H₂O will be in the starting material gas having CO as the main carbon source, the higher the yield of methanol is, but the CO conversion and the methanol yield are hardly affected even when the starting material gas contains about 1% of CO₂ and H₂O, respectively. However, when the starting material gas contains CO₂ and H₂O at a concentration higher than the above concentration, the CO conversion and the methanol yield decrease.

The process for producing methanol according to the present invention is presumably based on the following reaction (a case where the alcohol used in the reaction is an alcohol in which a hydroxyl group is bonded with a chained or an alicyclic hydrocarbon is shown as an example).

ROH + CO → HCOOR (6)

HCOOR + 2H₂ → CH₃OH + ROH (7)

(Wherein, R represents an alkyl group).

Accordingly, the starting materials for producing methanol are at least one of the following combinations of compounds; i.e., carbon monoxide and hydrogen, and carbon dioxide and hydrogen. Alcohols can be recovered and reused. According to the present invention, decrease of the catalytic activity is small even if a small amount of water or carbon dioxide is present in the starting material gas.

It should be noted that when the catalyst of the present invention which contains Cu, Mg, Na, and Pd in addition to an alkali metal formate salt is used in the liquid phase, part of the alkali metal formate salt or the entire salt, depending on the conditions, dissolves and functions as a catalyst, while the catalyst containing Cu, Mg, Na, and Pd functions as a solid catalyst. Hence, even if the above two components are separated in the reaction system, both exert their effects in the form of catalytic action. Accordingly, when preparing a catalyst, an alkali metal formate salt and a solid catalyst containing Cu, Mg, Na, and Pd can each be loaded to the reaction system individually, or the mixture thereof can be loaded to the reaction system to be used as the catalyst of the present invention.

### EXAMPLES

The present invention will be described in further detail using Examples 1 to 9 and Comparative Example 1. However, the present invention is not limited to these Examples. In addition, results from these Examples are summarized in a table shown below.

### [Example 1]

In an autoclave having a content volume of 50 ml, 2.5 mmol of potassium formate as well as 1 g of a Cu/MgO_{X}/Na₂CO₃ (18.7 mass%)/Pd(0.25 mass%) catalyst, in which 18.7 mass% of Na₂CO₃ and 0.25 mass% of Pd relative to Cu/MgO_{X} that was prepared from Cu(NO₃)₂·3H₂O and Mg(NO₃)₂·6H₂O as starting materials by coprecipitation method while maintaining the pH at 10.0 were sequentially impregnated therein and consequently loaded thereon, was added to 10 ml of ethanol as a solvent, synthesis gas (i.e., 32.40 vol% of CO, 64.58 vol% of hydrogen, and 3.02 vol% ofAr) was filled to 5MPa, and the reaction was performed at 160°C for 5 hours. The reaction products were analyzed by gas chromatography. The amount of methanol produced was 75.2 mmol and the amount of ethyl formate produced was 2.1 mmol. As compared with a Cu/MgO_{X}/Na₂CO₃ (18.7 mass%) which did not load Pd, described later in Comparative Example 1, the Cu/MgO_{X}/Na₂CO₃/Pd used above showed considerably higher catalytic activity.

### [Example 2]

The reaction was performed by the method described in Example 1 except that the reaction temperature was changed to 180°C. The amount of methanol produced was 56.4 mmol and the amount of ethyl formate produced was 1.1 mmol.

### [Example 3]

The reaction was performed by the method described in Example 1 except that the reaction temperature was changed to 140°C. The amount of methanol produced was 20.7 mmol and the amount of ethyl formate produced was 2.2 mmol.

### [Example 4]

The reaction was performed by the method described in Example 1 except that a Cu/MgO_{X}/Na₂CO₃ (18.7 mass%)/Pd (0.001 mass%) catalyst was added instead of the Cu/MgO_{X}/Na₂CO₃ (18.7 mass%)/Pd (0.25 mass%) catalyst. The amount of methanol produced was 53.1 mmol and the amount of ethyl formate produced was 1.8 mmol.

### [Example 5]

The reaction was performed by the method described in Example 1 except that a Cu/MgO_{X}/Na₂CO₃ (18.7 mass%)/Pd (0.005 mass%) catalyst was added instead of the Cu/MgO_{X}/Na₂CO₃ (18.7 mass%)/Pd (0.25 mass%) catalyst. The amount of methanol produced was 70.1 mmol and the amount of ethyl formate produced was 1.9 mmol.

### [Example 6]

The reaction was performed by the method described in Example 1 except that a Cu/MgO_{X}/Na₂CO₃ (18.7 mass%)/Pd (0.01 mass%) catalyst was added instead of the Cu/MgO_{X}/Na₂CO₃ (18.7 mass%)/Pd (0.25 mass%) catalyst. The amount of methanol produced was 81.9 mmol and the amount of ethyl formate produced was 2.2 mmol.

### [Example 7]

The reaction was performed by the method described in Example 1 except that a Cu/MgO_{X}/Na₂CO₃ (18.7 mass%)/Pd (0.025 mass%) catalyst was added instead of the Cu/MgO_{X}/Na₂CO₃ (18.7 mass%)/Pd (0.25 mass%) catalyst. The amount of methanol produced was 103.3 mmol and the amount of ethyl formate produced was 2.5 mmol.

### [Example 8]

The reaction was performed by the method described in Example 1 except that a Cu/MgO_{X}/Na₂CO₃ (18.7 mass%)/Pd (0.05 mass%) catalyst was added instead of the Cu/MgO_{X}/Na₂CO₃ (18.7 mass%)/Pd (0.25 mass%) catalyst. The amount of methanol produced was 101.5 mmol and the amount of ethyl formate produced was 2.3 mmol.

### [Example 9]

The reaction was performed by the method described in Example 1 except that a Cu/MgO_{X}/Na₂CO₃ (18.7 mass%)/Pd (0.1 mass%) catalyst was added instead of the Cu/MgO_{X}/Na₂CO₃ (18.7 mass%)/Pd (0.25 mass%) catalyst. The amount of methanol produced was 77.9 mmol and the amount of ethyl formate produced was 2.2 mmol.

### [Comparative Example 1]

The reaction was performed by the method described in Example 1 except that a Cu/MgO_{X}/Na₂CO₃ (18.7 mass%) catalyst which did not load Pd was added instead of the Cu/MgO_{X}/Na₂CO₃ (18.7 mass%)/Pd (0.25 mass%) catalyst. The amount of methanol produced was 42.1 mmol and the amount of ethyl formate produced was 2.3 mmol.

**[Table 1]**

| | Experimental features | Amount of methanol produced (mmol) | Amount of ethyl formate produced (mmol) |
|---|---|---|---|
| Example 1 | Temp: 160°C; potassium formate 2.5 mmol + Cu/MgOₓ/Na₂CO₃ (18.7 mass%)/Pd(0.25 mass%): 1 g | 75.2 | 2.1 |
| Example 2 | Temp: 180°C; potassium formate 2.5 mmol + Cu/MgOₓ/Na₂CO₃ (18.7 mass%)/Pd(0.25 mass%): 1 g | 56.4 | 1.1 |
| Example 3 | Temp: 140°C; potassium formate 2.5 mmol + Cu/MgOₓ/Na₂CO₃ (18.7 mass%)/Pd(0.25 mass%): 1 g | 20.7 | 2.2 |
| Example 4 | Temp: 160°C; potassium formate 2.5 mmol + Cu/MgOₓ/Na₂CO₃ (18.7 mass%)/Pd(0.001 mass%): 1 g | 53.1 | 1.8 |
| Example 5 | Temp: 160°C; potassium formate 2.5 mmol + Cu/MgOₓ/Na₂CO₃ (18.7 mass%)/Pd(0.005 mass%): 1 g | 70.1 | 1.9 |
| Example 6 | Temp: 160°C; potassium formate 2.5 mmol + Cu/MgOₓ/Na₂CO₃ (18.7 mass%)/Pd(0.01 mass%): 1 g | 81.9 | 2.2 |
| Example 7 | Temp: 160°C; potassium formate 2.5 mmol + Cu/MgOₓ/Na₂CO₃ (18.7 mass%)/Pd(0.025 mass%): 1 g | 103.3 | 2.5 |
| Example 8 | Temp: 160°C; potassium formate 2.5 mmol + Cu/MgOₓ/Na₂CO₃ (18.7 mass%)/Pd(0.05 mass%): 1 g | 101.5 | 2.3 |
| Example 9 | Temp: 160°C; potassium formate 2.5 mmol + Cu/MgOₓ/Na₂CO₃ (18.7 mass%)/Pd(0.1 mass%): 1 g | 77.9 | 2.2 |

In the liquid phase synthesis of methanol at low temperatures where an alkali metal formate salt and a hydrogenolysis catalyst are used, it is apparent from the above Examples and Comparative Example that the activity of Cu/MgO_{X}/Na₂CO₃, which is the hydrogenolysis catalyst, increases distinctively when loading a small amount of Pd. INDUSTRIAL APPLICABILITY

The present invention relates to a catalyst for methanol synthesis, in which methanol is synthesized via a formic ester and the reaction is carried out under the presence of a starting material gas, which contains hydrogen and at least either one of carbon monoxide and carbon dioxide, and an alcohol as a solvent, wherein the catalyst includes, in addition to an alkali metal formate salt, a catalyst containing Cu, Mg, Na, and Pd. When methanol is produced in a system where a catalyst containing Cu, Mg, Na, and Pd coexists with an alkali metal formate salt, under the presence of the starting material gas for synthesis containing hydrogen and at least either one of carbon monoxide and carbon dioxide, and a solvent alcohol, it is possible to synthesize methanol stably at high efficiency in a continuous reaction under low temperature and low pressure conditions. Moreover, it is possible to produce methanol at low cost since the extent of reductions in the catalytic activity remains low even when a small amount of water, carbon dioxide, or the like is mixed in the starting material gas for synthesis.

## Claims

1. A catalyst for methanol synthesis, where methanol is synthesized via a formic ester, which carries out a reaction under the presence of a starting material gas containing hydrogen and at least either one of carbon monoxide and carbon dioxide, and an alcohol as a solvent,
the catalyst comprising an alkali metal formate salt, and a catalyst containing Cu, Mg, Na, and Pd.

2. The catalyst for methanol synthesis according to Claim 1, wherein the alkali metal formate salt is potassium formate.

3. A catalyst for methanol synthesis, where methanol is synthesized via a formate ester, which carries out a reaction under the presence of a source gas containing hydrogen and at least either one of carbon monoxide and carbon dioxide, and an alcohol as a solvent,
the catalyst comprising Cu, Mg, Na, Pd, and an alkali metal-based catalyst which can change into an alkali metal formate salt.

4. The catalyst for methanol synthesis according to Claim 3, wherein the alkali metal-based catalyst which can change into an alkali metal formate salt is an alkali metal carbonate salt.

5. The catalyst for methanol synthesis according to any one of Claims 1 to 4, wherein said Na is loaded as a carbonate salt or a formate salt on a Cu/MgO solid catalyst.

6. The catalyst for methanol synthesis according to any one of Claims 1 to 5, wherein said Pd is loaded on a Cu/MgO solid catalyst.

7. The catalyst for methanol synthesis according to any one of Claims 1 to 6, wherein a loading amount of Pd is 0.001 to 1 mass% based on the catalyst containing Cu, Mg, Na, and Pd.

8. A method for producing a catalyst for methanol synthesis described in any one of Claims 5 to 7, the method comprising:
preparing the Cu/MgO solid catalyst; and
supporting Na and Pd on the solid catalyst.

9. A method for producing a catalyst for methanol synthesis described in any one of Claims 5 to 7, the method comprising:
preparing said Cu/MgO by coprecipitation method; and
loading Na and Pd on said Cu/MgO by impregnation method.

10. A method for producing a catalyst for methanol synthesis described in any one of Claims 5 to 7, the method comprising:
preparing said Cu/MgO by coprecipitation method while maintaining a constant pH within a range of 8 to 11.

11. A method for producing methanol, comprising:
reacting a starting material gas containing hydrogen and at least either one of carbon monoxide and carbon dioxide under the presence of the catalyst described in any one of Claims 1 to 7 and an alcohol, thereby producing a formic ester and methanol; and
hydrogenating the formic ester thus obtained to produce methanol.

12. A method for producing methanol comprising:
reacting a starting material gas containing hydrogen and at least either one of carbon monoxide and carbon dioxide under the presence of the catalyst described in any one of Claims 1 to 7 and an alcohol;
separating products obtained from a reaction system; and
hydrogenating the formic ester in the products by using a hydrogenolysis catalyst to produce methanol.

13. The method for producing methanol according to Claim 11 or 12, wherein an alkali metal-based catalyst which can change into an alkali metal formate salt during reaction is used instead of the alkali metal formate salt.

14. The method for producing methanol according to any one of Claims 11 to 13, wherein the alkali metal formate salt is potassium formate.

15. The method for producing methanol according to Claim 14, wherein the alkali metal-based catalyst which can change into an alkali metal formate salt during reaction is an alkali metal carbonate salt.

16. The method for producing methanol according to any one of Claims 11 to 15, wherein the alcohol is a primary alcohol.
